# EUROPÄISCHE PATENTSCHRIFT

(11) **EP 2 026 722 B1**
(45) Veröffentlichungstag und Bekanntmachung des Hinweises auf die Patenterteilung: **14.12.2011**
(21) Anmeldenummer: 07764635.4
(22) Anmeldetag: 13.06.2007
(51) Int. Cl.: A61F 5/37

(54) **SICHERHEITSBANDAGE MIT OBERSCHENKELMANSCHETTEN**
SAFETY BANDAGE WITH THIGH CUFFS
BANDAGE DE SÉCURITÉ AVEC MANCHONS POUR LES CUISSES

(30) Priorität: 14.06.2006 DE 202006009397 U
(43) Veröffentlichungstag der Anmeldung: 25.02.2009
(73) Patentinhaber: Sànchez, Alexander, 21266 Jesteburg (DE); Wysozki, Roman, 22763 Hamburg (DE)
(72) Erfinder: Sànchez, Alexander, 21266 Jesteburg (DE); Wysozki, Roman, 22763 Hamburg (DE)
(74) Vertreter: Richter, Werdermann, Gerbaulet & Hofmann
(86) Internationale Anmeldenummer: PCT/EP2007/005199
(87) Internationale Veröffentlichungsnummer: WO 2007/144155

(56) Entgegenhaltungen:
- DE-B3-102004 063 444
- DE-U1- 20 317 701
- US-A- 4 960 115
- US-A- 5 190 055

## Beschreibung

### Technisches Gebiet

Die Erfindung betrifft eine Sicherheitsbandage zur Begrenzung der Beweglichkeit eines auf einem Lager befindlichen Patienten bzw. zur Positionierung eines Patienten auf einem Lager mit einem Haltegurt, mit dem ein Leibgurt, der den Körper des Patienten im Bauchbereich umschließt, beweglich verbunden ist, und mit Oberschenkelmanschetten, die die Oberschenkel des Patienten umgreifen.

### Stand der Technik

Derartige Sicherheitsbandagen sind dem Fachmann in den unterschiedlichsten Ausführungsformen bekannt und geläufig und beispielsweise in der DE 296 01 701 U1 beschrieben.

Mit dem um den Bauchbereich des Patienten geschlungenen Leibgurt wird dessen Beweglichkeit soweit eingeschränkt, dass er das Lager, an dem der Leibgurt über einen Haltgurt befestigt ist, nicht entgegen dem Willen einer Aufsichtsperson verlassen kann. Um den Leibgurt relativ zum Körper des Patienten an diesem zu fixieren, ohne den Leibgurt zu stramm anziehen zu müssen, was beispielsweise zu einer Beeinträchtigung der Bauchatmung des Patienten führen könnte, ist es aus der DE 203 17 701 U1 bekannt, an dem Leibgurt bzw. der Sicherheitsbandage über quer zu diesem ausgerichtete Beinhalterungsgurte zusätzliche Oberschenkelmanschetten anzuordnen. Dabei wird jeder der beiden Oberschenkel des Patienten von einer diesem zugeordneten Oberschenkelmanschette umgriffen, die mit geeigneten Verschlussmitteln zum Einstellen des Oberschenkelumfangs des Patienten versehen sind. Derartige Oberschenkelmanschetten gewährleisten, dass der Patient sich weder bewusst noch unbewusst aus der Sicherheitsbandage bzw. deren Leibgurt nach unten herauswinden und das Lager, beispielsweise ein Bett oder eine speziell vorgesehene Krankenliege, verlassen kann. Zusätzlich kann ein Gurt vorgesehen sein, der quer zum Leibgurt durch den Schritt des Patienten verläuft. Dieser kann jedoch bei einem Versuch des Herauswindens aus dem Gurt zu einer Genitalquetschung des Patienten führen. Ebenso sind Selbststrangulationen der Patienten mit diesem Gurt nicht ausgeschlossen.

Bei derartigen Sicherheitsbandagen mit Beinhalterungs- und Oberschenkelmanschetten ist es jedoch nicht ausgeschlossen, dass besonders ein gelenkiger Patient die Knie zum Oberkörper hin anzieht, so dass der relative Abstand zwischen dem Leibgurt und den Oberschenkelmanschetten verringert ist. Bei angezogenen Knien kann so der Patient die Oberschenkelmanschetten über seine Knie abstreifen, da die Beinhalterungsgurte lose sind.

Aus der DE 203 17 701 ist eine Sicherheitsbandage zur Begrenzung der Beweglichkeit eines auf einem Lager liegenden Patienten bzw. zur Positionierung eines Patienten auf einem Lager bekannt, mit einem fest auf das Lager auflegbaren, sich über dieses erstreckenden Haltegurt, mit dem ein Leibgurt, der den Körper des Patienten im Bauchbereich umschlingt, beweglich verbunden ist, wobei für den Haltegurt und für den Leibgurt Verschlussmittel vorgesehen sind, wobei die Sicherheitsbandage über Mittel verfügt, die quer zum Leibgurt angeordnet sind und die über die Schultern legbar und zwischen den Beinen des Patienten hindurchführbar oder an den Beinen des Patienten festlegbar sind und die Mittel über Verschlussmittel zur Fixierung der Mittel in Relation zum Leibgurt verfügen können.

### Darstellung der Erfindung, Aufgabe, Lösung, Vorteile

Es ist daher Aufgabe der Erfindung, eine gattungsgemäße Sicherheitsbandage derart zu verbessern, dass ein Patient die Oberschenkelmanschetten nicht selbsttätig abstreifen kann, ohne ihn über Gebühr einzuengen, insbesondere soll ein seitliches Ziehen oder Drehen der Oberschenkelmanschetten, um diese abstreifen zu können, unterbunden werden.

Diese Aufgabe wird durch die Merkmale des Anspruchs 1 gelöst.

Der Grundgedanke der Erfindung ist es, dass zwischen den Beinhalterungsgurten eine materiale Fläche ausgebildet ist. Die Funktion einer das Gesäß haltenden materialen Fläche kann auch mit mehreren materialen Querstegen erreicht werden, wobei jedoch anzumerken ist, dass ein materialer Quersteg nicht ausreichend ist. Auf diese Weise wird verhindert, dass die Beinhalterungsgurte seitlich nach außen gezogen und so die den Beinhaltungsgurten jeweils zugeordneten Oberschenkelmanschetten über die Knie des Patienten von ihm abgestreift werden können. Zieht der Patient beispielsweise nur ein Bein an, um dessen Oberschenkelmanschette abzustreifen, so bewirken die materialen Querstege, dass dieses Anziehen oder Anwinkeln nur bis zu einem gewissen Grad möglich ist, da über die materialen Querstege oder die materiale Fläche auch das andere Bein über den Beinhalterungsgurt und die Oberschenkelmanschette in Richtung zum Körper hin mitgezogen wird. Werden aber beide Beine gleichzeitig angezogen, so dass die materialen Querstege oder die materiale Fläche nicht unter Zugspannung gesetzt werden, so ist ein Abstreifen der Oberschenkelmanschette nicht möglich, da durch die Beinhalterungsgurte die maximal mögliche Entfernung der Oberschenkelmanschette vom Leibgurt vorgegeben und deren Abstreifen verhindert ist. Bei angezogenen Beinen üben die materialen Querstege oder die materiale Fläche einen sanften Druck auf die Oberschenkel oder das Gesäß des Patienten aus, so dass dieser keine weiteren Versuche zum Abstreifen der Oberschenkelmanschette unternehmen wird. Mit den zusätzlichen materialen Querstegen ist gewährleistet, dass der Abstand der beiden Beinhalterungsgurte nicht über die durch die materialen Querstege oder die materiale Fläche vorgegebene Länge vergrößert werden kann, so dass der Patient beispielsweise nicht mit seinem Gesäß zwischen den Beinhalterungsgurten hindurchschlüpfen kann, um die Oberschenkelmanschette abzustreifen.

Die genaue Position und Breite der materialen Querstege bzw. deren Abstand zu den Oberschenkelmanschetten und zum Leibgurt kann vom Fachmann gewählt werden, um das Abstreifen wirkungsvoll zu unterbinden.

Das Material der Querstege und der Fläche entspricht den bekannten Materialien derartiger Sicherheitsbandagen und ist vorzugsweise das gleiche wie das Material der Leibgurte, der Beinhalterungsgurte oder der Oberschenkelmanschetten.

Der Vorteil der Erfindung besteht darin, dass mit den Oberschenkelmanschetten ein selbsttätiges Befreien des Patienten von der Sicherheitsbandage nicht möglich ist wobei die Oberschenkelmanschetten insbesondere keinen Druck auf die Genitalien des Patienten ausüben und Genitalquetschungen vermieden werden, da kein zusätzlicher Gurt durch den Schritt notwendig ist. Mit den zusätzlichen materialen Querstegen ist zudem verhindert, dass der Patient entweder nacheinander oder gleichzeitig die Oberschenkelmanschetten abstreifen könnte. Die Oberschenkelmanschetten verhindern auch, dass der Patient im Leibgurt nach unten rutscht und derart durch den Leibgurt ein zu großer Druck auf den Brustkasten des Patienten ausgeübt wird oder für den Patienten die Gefahr der Selbststrangulation besteht. Zudem behält der Patient ein relativ großes Maß an Bewegungsfreiheit, da eine sonst notwendige Diagonalfixierung zur Vermeidung der Selbststrangulation nicht vorgesehen ist.

Ein weiterer großer Vorteil dieser erfindungsgemäßen Sicherheitsbandage im Gegensatz zu Hosen, jackenartigen und schlafsackartigen Konstruktionen ist es, dass keine unnötige Erwärmung bzw. Überhitzung des Patienten vorkommt, da tatsächlich nur Gurte angebracht werden, die genügend Belüftungsfläche lassen.

Vorteilhafte Ausgestaltungen der Erfindung sind in den Unteransprüchen gekennzeichnet.

Bei einer bevorzugten Ausführungsform sind an einer Oberseite der Sicherheitsbandage bzw. an deren Leibgurt zwei zusätzliche Längsgurte angeordnet, die jeweils mit den Oberschenkelmanschetten verbunden sind. Diese zusätzlichen Längsgurte sind ebenfalls im Wesentlichen senkrecht zum Leibgurt ausgerichtet und verbinden diesen im angelegten Zustand der Sicherheitsbandage an der Oberseite der Oberschenkel des Patienten verlaufend mit den Oberschenkelmanschetten. Diese Längsgurte unterbinden eine Abstandsvergrößerung der Oberschenkelmanschetten zum Leibgurt an der Oberseite, um deren relativen Abstand zueinander aufrecht zu erhalten.

Nach einer besonders vorteilhaften Ausführungsform ist für die zusätzlichen Längsgurte ein weiterer Quersteg vorgesehen, der an den Längsgurten befestigt, beispielsweise vernäht ist, und im angelegten Zustand der Sicherheitsbandage an den Außenseiten der Oberschenkel des Patienten anliegt und diese von hinten durchgehend umlaufend umgreift. Zusätzlich ist der zweite Quersteg mit den Beinhaltungsgurten verbunden. An der Oberseite zwischen den Längsgurten ist der zweite Quersteg unterbrochen. Somit ist vermieden, dass die obenseitigen Längsgurte zur Mitte hin, also zur Symmetrieebene der Sicherheitsbandage, aufeinander zugezogen oder bewegt werden können, um den Abstand zwischen den Oberschenkelmanschetten und dem Leibgurt in unerwünschter Weise zu verringern. Dies wird dadurch vermieden, dass der durchgehende Quersteg an der Rückseite der Oberschenkel des Patienten verläuft und so die Längsgurte nicht zusammengezogen werden können. Ein seitliches Ausrücken eines Längsgurts nach innen ist durch den durch den zweiten Quersteg auf den anderen Längsgurt übertragenem Zug unterbunden. Außerdem sind die Beinhalterungsgurte mit dem zweiten Quersteg zusätzlich in ihrer Lage fixiert. Der durchgehend verlaufende zweite Quersteg übt beim gemeinsamen Anziehen der Oberschenkel ebenfalls einen sanften Druck auf diese aus, so dass der Patient die Oberschenkelmanschetten weder einzeln noch gemeinsam abstreifen kann.

Zusätzlich können an der Sicherheitsbandage dem Fachmann bekannte Schultergurte vorgesehen sein, die ebenfalls im Wesentlichen quer zum Leibgurt ausgerichtet oder sich kreuzend angeordnet und über die Schultern des Patienten geführt und am Leibgurt befestigt sind. Gemeinsam mit derartigen Schulterhaltungen ist ein selbsttätiges Herauswinden des Patienten aus der Sicherheitsbandage praktisch ausgeschlossen. Das Abstreifen der Oberschenkelmanschetten wird durch die am Leibgurt fest oder lösbar angebrachten Schultergurte wirksam verhindert. Dem Patienten ist es nicht möglich, den Leibgurt und somit die daran befestigten Oberschenkelmanschetten nach unten zu ziehen und sodann abzustreifen. Der Versuch, mittels Anwinkeln der Beine und/oder Krümmens des Rückens die Oberschenkelmanschetten abzustreifen, wird mittels des von den Schultergurten ausgehenden Zuges über Leibgurt und Beinhaltungsgurte unterbunden. Der Sicherheitsbandage liegt also ein Mechanismus derart zugrunde, dass beim Versuch, mittels Abwinkeln der Beine und/oder Krümmens des Rückens den Abstand der Oberschenkelmanschetten zum Knie zu vermindern, der Abstand mittels von den Schultergurten über Rücken und Gesäß geleiteten Zuges wiederum quasi selbstregulierend vergrößert wird, was das Abstreifen der Oberschenkelmanschetten wirksam unterbindet. Der Längsgurt und die Querstege bzw. die materiale Fläche verhindern dabei das Ausweichen bzw. die Umgehung dieses Mechanismus, so dass der Patient mittels der somit nicht entfernbaren Oberschenkelmanschetten ohne Gefahr der Strangulation oder Genitalquetschung am Herausrutschen nach unten und mittels der Schultergurte nach oben gehindert wird und schlussendlich in der Sicherheitsbandage verbleibt. Es versteht sich, dass die Schulterhalterungen entweder lösbar, z. B. mit Schlaufen, am Leibgurt bzw. Haltegurt befestigbar sind, oder fest daran angeordnet sind, vorzugsweise mit diesem vernäht. Derartige Schulterhalterungen können auch als Nachrüstsatz für bereits vorhandene Leibgurte ausgebildet sein, die zusätzlich an diesem befestigt werden, um die Sicherheit zu erhöhen.

Zur Erhöhung des Tragekomforts der Sicherheitsbandage und um Druckstellen zu vermeiden sind die materiale Fläche und die materialen Querstege aus Stoff oder Gewebe. In gleicher Weise kann ein Netz aus hierfür geeigneten Materialien die Funktion der Querstege übernehmen. Im Rahmen der Erfindung ist es auch mit umfasst, dass der erste und der zweite Quersteg und gegebenenfalls die Oberschenkelmanschetten einstückig miteinander und durchgehend und flächig ausgebildet sind. In diesem Fall würde eine derartige flächige Ausführung beim Anziehen der Oberschenkel einen Druck auf das Gesäß und die Rückseite der Oberschenkel des Patienten ausüben, so dass ein selbsttätiges Abstreifen der Oberschenkelmanschetten ausgeschlossen ist. Hierbei bilden der Leibgurt, eine im Folgenden beschriebene Schulterhalterung sowie der Quersteg oder das Netz zwischen den Beinhalterungsgurten eine Einheit. Zieht der Patient ein oder beide Knie an so wird über das Netz in Verbindung mit den Schultergurten ein Zug auf die Oberschenkelmanschetten in Richtung zum Oberkörper des Patienten hin ausgeübt, so dass er diese nicht über die Knie abstreifen kann.

Um bereits vorhandene Sicherheitsbandagen mit Oberschenkelmanschetten nachzurüsten, die gegen ein Abstreifen gesichert sind, sind die Beinhalterungsgurte am Leibgurt oder am Haltegurt und gegebenenfalls die Längsgurte an der Oberseite der Sicherheitsbandage lösbar am Leibgurt befestigt. Hierfür können sämtliche im Stand der Technik bekannten Befestigungsmittel wie Segelösen mit Magnetknöpfen, Gurtschlösser, Klettverschlüsse, Laschen und Schlaufen oder dergleichen vom Fachmann verwendet werden. Somit ist es möglich, eine Sicherheitsbandage, die im Wesentlichen aus einem Leibgurt besteht, mit den Oberschenkelmanschetten nachzurüsten, wobei durch die zusätzlichen Querstege deren unerwünschtes Abstreifen unterbunden ist. Ein derartiger Nachrüstsatz kann mit einem vorhandenen Leibgurt auch fest vernäht werden.

Es ist von Vorteil, dass die Beinhalterungsgurte und die Schultergurte sowie die Längsgurte einstückig ausgebildet sind, wobei die einstückig ausgebildeten Gurte in eine Durchstecktasche, die sich an dem Leibgurt oder Haltegurt befindet, durchsteckbar sind. Auf diese Weise kann der Abstand zwischen Leibgurt und Oberschenkelmanschetten variabel gestaltet werden. Dazu dient auch, dass der einstückig ausgebildete Gurt Segelösen und/oder Taschen und/oder Schlaufen aufweist. Die einstückig ausgebildeten Gurte werden dann durch die Durchstecktasche gezogen und mit Schlössern an den Enden des Leibgurtes sowie der Oberschenkelmanschetten befestigt. Dieses kann in der Weise erfolgen, dass die einstückig ausgebildeten Gurte über den Leibgurt parallel zu den Oberschenkelmanschetten geführt werden und sich über dem Leibgurt kreuzen.

Vorzugsweise sind die Enden an den Oberschenkelmanschetten, die als Betthalterung dienen, diagonal mit den Enden des Haltegurtes fest oder lösbar verbindbar. Auch auf diese Weise wird das Drehen des Patienten um die vertikale Achse verhindert.

Vorzugsweise ist der Leibgurt mit einer Schulterhalterung fest verbunden, wobei die Schulterhalterung mit zwei Schultergurten versehen ist, die über die Schultern des Patienten verlaufen, sowie im mittleren Bereich eines Taillengurtes mit dem Taillengurt verbunden sind, wobei der Taillengurt die Taille des Patienten umgreifen kann und beide Enden des Taillengurtes mit jeweils beiden Schultergurten - diese wiederum miteinander verbindend - lösbar miteinander verbunden sind, sowie Gurtschlösser, die mit einer mit geeigneten Verschlussmitteln zu öffnenden Verriegelungsposition versehen sind, die Enden des Taillengurtes auf die Schultergurte jeweils paarweise miteinander verbunden sind. Auf diese Weise wird erreicht, dass gegenüber am Leibgurt bzw. Haltegurt lösbar angebrachten Schulterhalterung ein festerer Sitz der Schulterhalterung am Leibgurt bzw. Haltegurt gegeben ist. Auch kann eine nach dem Stand der Technik befindliche Schulterhalterung fest oder lösbar mit dem Leib- oder Haltegurt verbunden werden.

Eine weitere Ausgestaltung der Erfindung sieht vor, dass mindestens ein zusätzlicher Quersteg zwischen den beiden im Wesentlichen quer zum Leibgurt angeordneten Schultergurten vorgesehen ist, wobei der Quersteg im Wesentlichen parallel zum Leibgurt ausgerichtet ist. Dieser zusätzliche Quersteg verhindert, dass die Schultergurte seitlich ausgerückt und über seine Schulter vom Patienten abgestreift werden können, da beim seitlichen Verschieben eines der Schultergurte über den Quersteg ein Zug auf den anderen Schultergurt übertragen und dieser in Richtung des ersten Schultergurts gezogen wird, so dass dieser beispielsweise am Hals des Patienten anstößt und ein weiteres seitliches Verschieben des ersten Schultergurts verhindert ist. Ebenso ist es durch den Quersteg unterbunden, dass beide Schultergurte gleichzeitig zur Seite oder nach außen hin auseinander gezogen werden. Mit dem zusätzlichen Quersteg ist gewährleistet, dass der Abstand der beiden Schultergurte nicht über die durch den Quersteg vorgegebene Länge vergrößert werden kann, so dass der Patient beispielsweise nicht seine Schulter aus einem der Gurte herauswinden kann, um die Schultergurte abzustreifen.

Im Rahmen der Erfindung ist es mit umfasst, dass die beiden Schultergurte im Wesentlichen senkrecht zum Leibgurt und parallel zueinander ausgerichtet sind sowie eine sich überkreuzend verlaufende Anordnung der Schultergurte.

Die genaue Position, das heißt der Abstand zum Leibgurt, und die Breite des Querstegs kann vom Fachmann ausgewählt werden, um das Abstreifen der Schultergurte wirkungsvoll zu unterbinden.

Es versteht sich, dass auch mehr als ein Quersteg, die jeweils im Wesentlichen quer zum Leibgurt ausgerichtet sind, zwischen den ungefähr parallel zueinander angeordneten Schultergurten vorgesehen sein können. Beispielsweise kann auch auf der Vorderseite der Sicherheitsbandage ungefähr im Brustbereich des Patienten ein weiterer Quersteg vorgesehen sein, der zum leichteren Anlegen der Sicherheitsbandage in vorteilhafter Weise zumindest an einem Schultergurt lösbar befestigbar ist. Das Material des Querstegs entspricht den bekannten Materialien derartiger Sicherheitsbandagen und ist vorzugsweise das gleiche wie das Material der Leibgurte oder der Schultergurte.

Der Vorteil dieser Ausführungsform besteht darin, dass mit den Schultergurten ein selbsttätiges Befreien des Patienten von der Sicherheitsbandage wirkungsvoll unterbunden ist wobei seine Bewegungsfreiheit durch die zusätzlichen Schultergurte, die untereinander mit einem Quersteg verbunden sind, nur unwesentlich eingeschränkt ist.

In einer weiteren Ausführungsform sind die Schultergurte an einem zusätzlichen Taillengurt angeordnet, der seinerseits im Wesentlichen parallel zum Leibgurt ausgerichtet ist. Der Taillengurt ist zur Umschlingung der Taille des Patienten vorgesehen und kann mit geeigneten Verschlussmitteln auf den Taillenumfang des Patienten eingestellt werden. Die Schultergurte sind vorzugsweise an der Rück- oder Unterseite der Sicherheitsbandage an dem Taillengurt befestigt und werden von hinten über die Schultern des Patienten geführt und an der Vorderseite entweder am Taillengurt oder am Leibgurt in geeigneter Weise befestigt. Insbesondere ist vorgeschlagen, dass am Leibgurt Schlaufen vorgesehen sind, durch die der zusätzliche Taillengurt hindurchgeführt ist, so dass der Taillengurt relativ zum Leibgurt beweglich ist bzw. seitlich geringfügig verschoben werden kann.

In einer alternativen Ausführungsform sind die Schultergurte, die im Wesentlichen parallel zueinander ausgerichtet sind, unmittelbar am Leibgurt oder dem Haltegurt befestigt und werden über die Schultern des Patienten geführt und mit geeigneten Verschlussmitteln an der Vorderseite des Leibgurts festgelegt.

Bei dieser Ausführungsform kann an den Schultergurten auch ein weiterer Brustgurt vorgesehen sein, der auf der Rückseite des Körpers des Patienten an den Schultergurten befestigt ist und im Wesentlichen quer zum Leibgurt ausgerichtet ist. Dieser Brustgurt umgreift den Brustbereich des Patienten und er kann auf der Vorderseite des Patienten an den über die Schultern geführten Schultergurten beispielsweise an daran angeformten Laschen oder Schlaufen ebenfalls festgelegt werden.

Bevorzugt sind der oder die Querstege zwischen den Schultergurten flächig ausgeführt, um derart eine große Auflagefläche zu erreichen, damit durch die Sicherheitsbandage keine Druckstellen auf der Haut des Patienten erzeugt werden. Dabei können die Querstege flächig in Form eines Stoffes oder Gewebes aus einem für Sicherheitsbandagen bekannten Material ausgeführt sein oder als Netz aus einem hierfür geeigneten Material.

Um bereits vorhandene Sicherheitsbandagen mit Schultergurten nachzurüsten, die gegen ein Abstreifen gesichert sind, sind die Schultergurte am Leibgurt oder am Haltegurt lösbar befestigt. Hierfür können sämtliche im Stand der Technik bekannten Befestigungsmittel wie Segelösen mit Magnetknöpfen, Gurtschlösser, Klettverschlüsse, Laschen und Schlaufen oder dergleichen vom Fachmann verwendet werden. Somit ist es möglich, eine Sicherheitsbandage, die im Wesentlichen aus einem Leibgurt besteht, mit den Schultergurten nachzurüsten, wobei durch die zusätzlichen Querstege deren unerwünschtes Abstreifen unterbunden ist. Ein derartiger Nachrüstsatz kann mit einem vorhandenen Leibgurt auch fest vernäht werden.

Es ist von Vorteil, dass die beiden Halterungsgurte an einem Fußende des Bettrahmens lösbar befestigbar sind. Auch ist es im Rahmen der Erfindung möglich, dass mindestens ein Verlängerungsgurt am Quersteg und/oder an den Beinhalterungsgurten und/oder Haltegurt und/oder Leibgurt befestigt sind und an einem Fußende des Bettrahmens lösbar befestigbar sind. Eine praktikable Variante der Erfindung sieht zudem vor, dass an den/dem Schultergurten und/oder Haltegurt und/oder Leibgurt und/oder Querstegen Verlängerungsgurte befestigbar sind, die am Kopfende des Bettrahmens lösbar befestigbar sind.

### Kurze Beschreibung der Zeichnungen

Nachstehend werden Ausführungsbeispiele der Erfindung anhand der Zeichnungen näher erläutert. Es zeigen in perspektivischer Darstellung:
- Fig. 1:: eine Sicherheitsbandage;
- Fig. 2:: eine Schulterhalterung, die mit der erfindungsgemäßen Sicherheitsbandage venvendbar ist;
- Fig. 3:: eine weitere Schulterhalterung; und
- Fig.4:: eine weitere Ausführungsform der erfindungsgemäßen Sicherheitsbandage.

### Bester Weg zur Ausführung der Erfindung

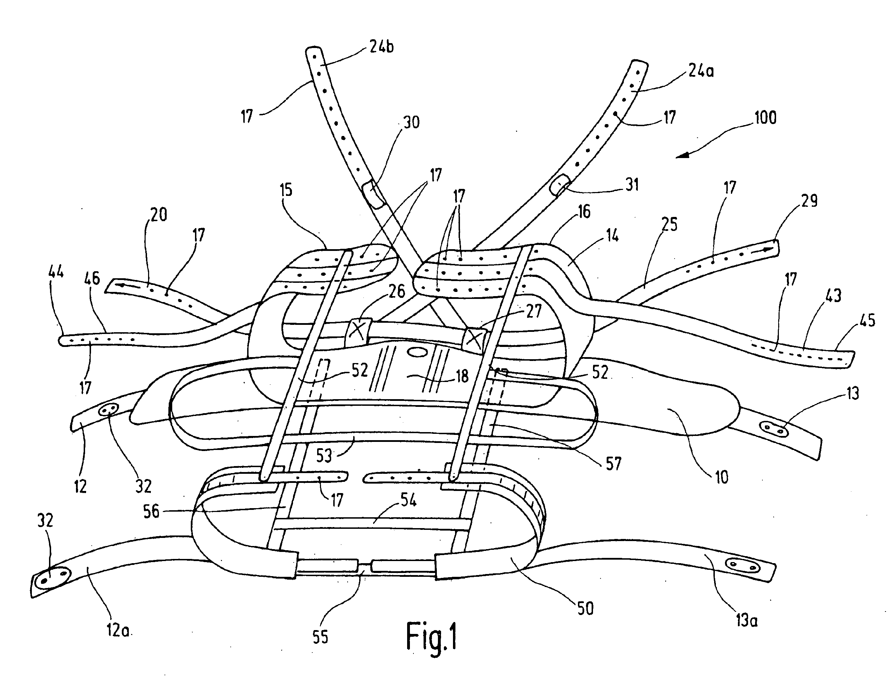

Die in Figur 1 dargestellte Sicherheitsbandage 100 besteht im Wesentlichen aus einem Haltegurt 10, der unmittelbar auf einer hier nicht dargestellten Unterlage, diese überquerend, aufgelegt und mittels an den Enden 12, 13 vorgesehenen Befestigungsmitteln wie z. B. Ösen 32 auf der Unterlage befestigt ist. Weiterhin besitzt die Sicherheitsbandage 100 einen Leibgurt 14, der mit dem Haltegurt 10 in ebenfalls nicht dargestellter Weise beweglich verbunden ist. Die Enden 15, 16 des Leibgurts 14 weisen jeweils drei Reihen von Segelösen 17 auf, die in Längsrichtung der Enden 15, 16 parallel angeordnet sind. Prinzipiell können auch nur eine oder zwei Reihen von Segelösen 17 vorgesehen sein. Mittig besitzt der Leibgurt 14 eine Durchstecktasche 18, durch die quer zum Leibgurt 14 ein mehrgliedriger Gurt entsprechend der DE 203 17 701 U1 gesteckt sein kann, dieser ist jedoch bei der erfindungsgemäßen Ausgestaltung der Sicherheitsbandage 100 nicht mehr notwendig.

Zusätzlich können an dem Leibgurt 14 weitere Befestigungsgurte 43, 44 vorgesehen sein, deren freien Enden 45, 46 jeweils mit Segelösen 17 zur Befestigung beispielsweise an der Unterlage oder an einem Bettgestell versehen sind. Die Befestigungsgurte 43, 44 können auch lösbar am Leibgurt 14 befestigt sein.

Zur Umgreifung der Schultern eines Patienten sind sich überkreuzende Schultergurte 24a, 24b mit Segelösen 17 an ihren freien Enden an einem zusätzlichen Gurt 25 angeordnet. Der Gurt 25, der im Wesentlichen parallel zum Leibgurt 14 verläuft, ist durch Schlaufen 26, 27 am Leibgurt 14 hindurchgeführt und verfügt an seinen freien Enden 28, 29 ebenfalls über Segelösen 17, um den Gurt 25 um die Taille des Patienten zu schlingen und festzulegen. Dabei kann der Gurt 25 durch eine Schlaufe 30, die an dem Abschnitt 24b angeordnet ist, hindurchgeführt werden. Zur Handhabung beispielsweise von Magnetverschlüssen am Gurt 25 sind ist am Abschnitt 24a eine Tasche 31 angeordnet.

Zusätzlich ist die Sicherheitsbandage 100 mit zwei Oberschenkelmanschetten 50 mit breiten manschettenartigen Abschnitten versehen, an deren einem freien und schmaleren Ende ebenfalls Segelösen 17 angeordnet sind, um diesen Abschnitt des Oberschenkelmanschetten 50 durch einen nicht dargestellten Metallring am anderen freien Ende hindurchzuschlaufen, um einen Oberschenkel des Patienten vollständig zu umschließen. Das Festlegen der Segelösen 17 erfolgt beispielsweise mit Magnetknöpfen oder mit sonstigen bekannten Verbindungsmitteln. Die Oberschenkelmanschetten 50 sind jeweils an einem Beinhalterungsgurt 56, 57 angeordnet, die im Wesentlichen senkrecht zum Leibgurt 14 ausgerichtet und an diesem befestigt sind. Zwischen den Beinhalterungsgurten 56, 57 ist ein Quersteg 54 angeordnet, um ein unabhängiges Anziehen oder Anwinkeln nur eines Beins oder Oberschenkels des Patienten zu unterbinden. Somit ist es ausgeschlossen, dass eine Oberschenkelmanschette 50 bei an die Brust des Patienten angezogenem Knie von diesem selbsttätig vom Oberschenkel über das Knie und den Unterschenkel abgestreift wird. An den Oberschenkelgurten 50 können auch weitere Enden 12a, 13a mit Ösen 32, entsprechend dem Haltegurt 10, zur Befestigung auf dem Lager vorgesehen sein.

Zusätzlich sind an der Oberseite der Sicherheitsbandage 100 weitere Längssgurte 52 vorgesehen, die im Wesentlichen vom Leibgurt 14 jeweils zu einer Oberschenkelmanschette 50 verlaufen und ebenfalls quer zum Leibgurt 14 ausgerichtet sind. Die Längsgurte 52 sind durchgehend um die Rückseite der Sicherheitsbandage 100 umlaufend mit einem zweiten Quersteg 53 verbunden sind. Somit ist es vermieden, dass die Längsgurte 52, die ebenfalls im Wesentlichen senkrecht zum Leibgurt 14 ausgerichtet sind, z. B. zur Mitte der Sicherheitsbandage 100 hin zusammengezogen und somit eine Oberschenkelmanschette 50 ungewünscht abgestreift werden kann. Zusätzlich ist im Bereich der Oberschenkelmanschette 50 ein dritter Quersteg 55 vorgesehen.

Die Oberschenkelmanschetten 50 sowie die Querstege 53, 54, 55 sind derart dimensioniert, dass sich keine Druckstellen auf der Haut des Patienten bilden. Zur Verbindung der verschiedenen Gurte und Stege miteinander sind diese jeweils an den Kontaktstellen vernäht und/oder verklebt oder mit sonstigen für Sicherheitsbandagen 100 bekannten Verbindungsmitteln miteinander verbunden.

Um eine Sicherheitsbandage 100 mit Oberschenkelmanschetten 50 nachzurüsten sind die Längsgurte 52 sowie die Beinhalterungsgurte 56, 57 lösbar am Leibgurt 14 bzw. dem Haltegurt 10 befestigt, beispielsweise an den Segelösen 17 des Leibgurts 14, und können auch als Nachrüstsatz für eine bereits vorhandene Sicherheitsbandage 100 angeboten werden.

Die Querstege 53, 54, 55 können auch flächig oder netzförmig ausgebildet sein bzw. sind sie derart dimensioniert und angeordnet, dass sie gemeinsam die Funktionalität einer flächigen Form erfüllen, um derart beim Anziehen der Beine oder der Oberschenkel einen sanften Druck auf Gesäß und die Rückseite der Oberschenkel des Patienten auszuüben, so dass dieser die Beine nicht anziehen kann, da gleichzeitig die sich überkreuzenden Abschnitte 24a, 24b eine Zugkraft in Gegenrichtung auf den Leibgurt 14 bzw. die Oberschenkelmanschette 50 ausüben. Somit ist die Ausbildung von Druckstellen wirkungsvoll vermieden. Die Oberschenkelmanschetten 50 können z. B. auf den Quersteg 55 aufgenäht sein.

Die Funktion des weiteren Querstegs 53 besteht vornehmlich darin, die Beinhalterungsgurte 56, 57 bzw. die dazwischen befindliche materiale Fläche mit den Längsgurten 52 zu verbinden, so dass ein seitliches Ziehen oder Drehen der Oberschenkelmanschetten 50, um diese Abstreifen zu können, unterbunden wird. Wenn die Funktionen der das Gesäß haltenden materialen Fläche mit Querstegen 54, 55 realisiert ist, können die Verbindungsgurte zwischen den Beinhalterungsgurten 56, 57 und Längsgurten 52 mit einem Gurt 53 realisiert werden, der gleichzeitig als Quergurt dient.

Das Abstreifen der Oberschenkelmanschetten 50 mittels am Leibgurt 14 fest oder lösbar angebrachten Schultergurten 24a, 24b kann wirksam dadurch verhindert werden, dass der Patient den Leibgurt 14 und somit die daran befestigten Oberschenkelmanschetten 50 nicht nach unten ziehen und diese sodann abstreifen kann. Somit wird der Versuch, mittels eines Anwinkelns der Beine und/oder Krümmens des Rückens die Oberschenkelmanschetten abzustreifen, durch den von den Schultergurten 24a, 24b aufgehenden Zug über Leibgurt und Beinhalterungsgurte unterbunden. Dazu trägt auch bei, dass der Längsgurt 52 sowie der zweite Quersteg 53 sowie der erste Quersteg und der weitere Quersteg 55 bzw. die materiale Fläche das Ausweichen des beschriebenen Mechanismus verhindert, so dass der Patient mit den nicht entfernbaren Oberschenkelmanschetten ohne Gefahr der Strangulation oder Genitalquetschung am Herausrutschen nach unten und mittels der Schultergurte 24a, 24b nach oben gehindert wird und schlussendlich in der Sicherheitsbandage verbleibt. Die Funktionsweise des Mechanismus liegt also darin, beim Versuch mittels eines Anwinkelns der Beine und/oder Krümmens des Rückens den Abstand der Oberschenkelmanschette 50 zum Knie zu verhindern und mittels des von den Schultergurten 24a, 24b über Rücken und Gesäß geleiteten Zuges den Abstand quasi selbstregulierend zu vergrößern, mit der Folge, dass das Abstreifen der Oberschenkelmanschetten wirksam unterbunden wird.

Weiterhin kann der Abstand zwischen Leibgurt 14 und Oberschenkelmanschetten 50 dadurch variabel gestaltet werden, dass am Leibgurt 14 eine Durchstecktasche 18 angebracht ist, durch die die Beinhalterungen 56, 57 gezogen werden. Letztere sind jedoch um die Längen des Leibgurtes 14, der Schultergurte 24a, 24b und die Längsgurte 52 verlängert und entsprechend mit Ösen und Schlaufen ausgestattet, so dass sie die Funktion dieser Gurte übernehmen. Diese nun einstückig ausgestalteten Gurte werden also durch die Durchstecktasche 18 gezogen und mit Schlössern an den Enden 15, 16 des Leibgurtes 14 sowie der Oberschenkelmanschetten 50 befestigt. Dies erfolgt derart, dass die einstückig ausgestalteten Gurte über den Leibgurt 14 parallel zu den Oberschenkelmanschetten 50 geführt werden und sich über dem Leibgurt 14 kreuzen. Damit die Gurte nicht über die Schultern abgestreift werden können, sind sie entsprechend den Schulterhalterungen in Höhe der Schulterblätter zusammengenäht und unterhalb der Achsel in unterschiedlichen Höhen mit Schlaufen versehen, damit der Taillengurt 28, 29 durchgesteckt und auf der Vorderseite mit Schlössern mit den einstückig ausgestalteten Gurten verbunden werden kann. Die einstückig ausgebildeten Gurte können dabei fest mit dem Leibgurt 14 vernäht sein.

Auch können (nicht gezeigt) die Enden 12a, 13a, d. h. die Betthalterungen an den Oberschenkelmanschetten, diagonal mit den Enden 12, 13 verbunden werden. Auf diese Weise wird das Drehen des Patienten um die vertikale Achse verhindert. Des Weiteren ist der Abstand zwischen Leibgurt 14 und Oberschenkelmanschette 50 so kurz ausgeführt, dass der Gurt 53 nicht erforderlich sein braucht (nicht gezeigt). Die Enden 12a, 13a können auch als Bettverbindungsgurt zur Befestigung am Lager ausgeführt sein (nicht gezeigt).

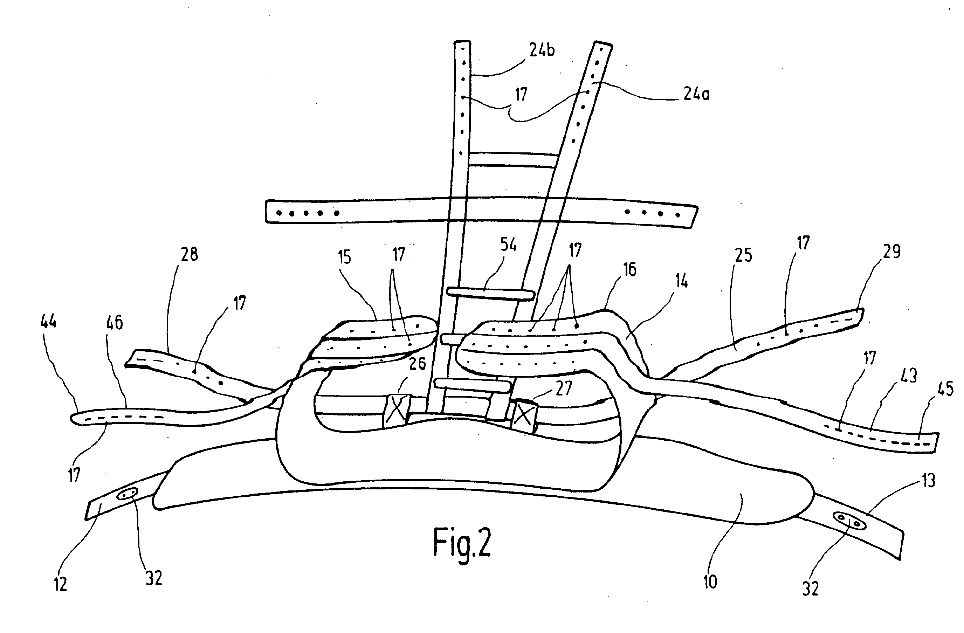

Die in Figur 2 ohne Beinhalterungen 56, 57 dargestellte Ausführungsform der Sicherheitsbandage 100 besteht im Wesentlichen aus einem Haltegurt 10, der unmittelbar auf einer hier nicht dargestellten Unterlage, diese überquerend, aufgelegt und mittels an den Enden 12, 13 vorgesehenen Befestigungsmitteln wie z. B. Ösen 32 auf der Unterlage befestigt ist. Weiterhin besitzt die Sicherheitsbandage 100 einen Leibgurt 14, der mit dem Haltegurt 10 in ebenfalls nicht dargestellter Weise beweglich verbunden ist. Die Enden 15, 16 des Leibgurts 14 weisen jeweils drei Reihen von Segelösen 17 auf, die in Längsrichtung der Enden 15, 16 parallel angeordnet sind. Prinzipiell können auch nur eine oder zwei Reihen von Segelösen 17 vorgesehen sein. Mit den Segelösen 17 wird der Leibgurt 14 auf den Bauchumfang des Patienten eingestellt und festgelegt.

Zusätzlich können an dem Leibgurt 14 weitere Befestigungsgurte 43, 44 als Seitenbefestigungen vorgesehen sein, deren freien Enden 45, 46, jeweils mit Segelösen 17 zur Befestigung beispielsweise an der Unterlage oder an einem Bettgestell versehen sind. Die Befestigungsgurte 43, 44 können auch lösbar am Leibgurt 14 befestigt sein.

Zur Umgreifung der Taille eines Patienten ist ein Taillengurt 25 an der Sicherheitsbandage 100 vorgesehen, der im Wesentlichen parallel zum Leibgurt 14 verläuft. Der Taillengurt 25 ist durch Schlaufen 26, 27 am Leibgurt 14 hindurchgeführt und verfügt an seinen freien Enden 28, 29 ebenfalls über Segelösen 17, um den Taillengurt 25 um die Taille des Patienten zu schlingen und beispielsweise mit Magnetknöpfen festzulegen.

An dem Taillengurt 25 sind zwei Schultergurte 24a, 24b befestigt, beispielsweise vernäht und/oder verklebt, die im Wesentlichen je nach Ausführungsform parallel oder über Kreuz zueinander ausgerichtet sind und über die Schultern eines Patienten geführt werden. Die Schultergurte 24a, 24b sind an ihren freien Enden ebenfalls mit Segelösen 17 versehen, um die Schultergurte 24a, 24b entweder am Taillengurt 25 oder am Leibgurt 14 an weiteren Segelösen 17 mit geeigneten Verbindungsmitteln festzulegen.

Um ein unerwünschtes Abstreifen der Schultergurte 24a, 24b durch den Patienten über seine Schultern zu unterbinden sind zwischen den Schultergurten 24a, 24b ein oder mehrere Querstege 54 vorgesehen, die im Wesentlichen parallel zum Leibgurt 14 ausgerichtet sind. Der oder die Querstege 54 verhindern, dass die Schultergurte 24a, 24b vom Patienten auseinander gezogen werden, um diese über die Schultern abzustreifen.

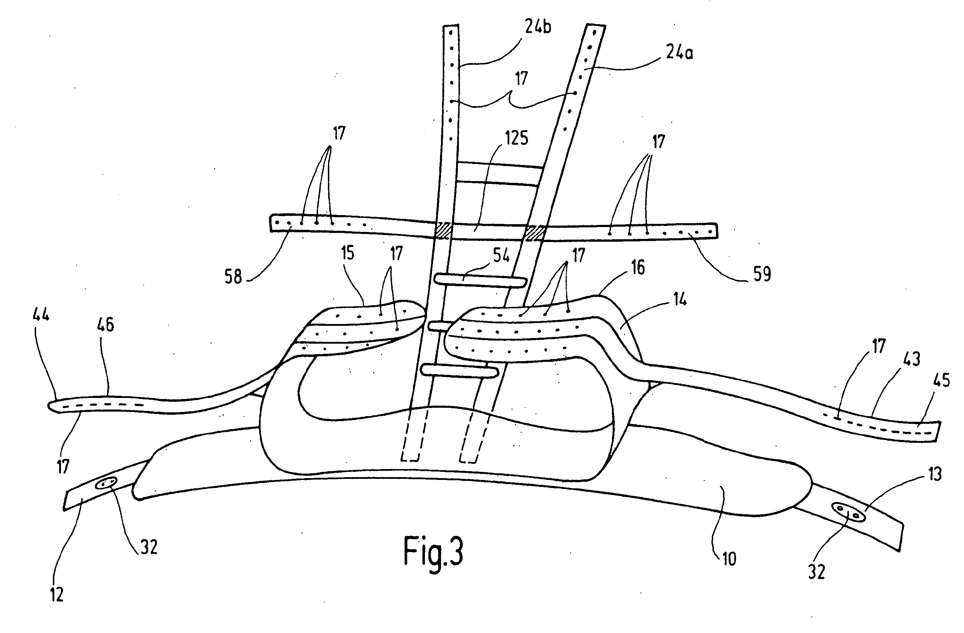

Bei der in Figur 3 ohne Beinhalterungen 56, 57 dargestellten alternativen Ausführungsform sind die Schultergurte 24a, 24b unmittelbar am Leibgurt 14 bzw. am Haltegurt 10 angeordnet und insbesondere lösbar befestigt. Die Schultergurte 24a, 24b sind hier ebenfalls mit Querstegen 54 miteinander verbunden, um ein seitliches Auseinanderziehen der Schultergurte 24a, 24b zu unterbinden. Zusätzlich ist an den Schultergurten 24a, 24b ein weiterer Brustgurt angeordnet, um den Brustbereich eines Patienten zu umschlingen. Auch dieser Brustgurt 60 wirkt als weiterer Quersteg 54. An den freien Enden 128, 129 des weiteren Brustgurts 125 sind ebenfalls Segelösen 17 zur Festlegung des Brustgurts 60 vorgesehen. Dabei kann der Brustgurt 60 auch durch hier nicht dargestellte Schlaufen an den Schultergurten 24a, 24b vor der Brust des Patienten hindurchgeführt sein.

Die Schultergurte 24a, 24b, die Brustgurte 60 und Taillengurte 25 sowie die Querstege 54 sind derart dimensioniert, dass sich keine Druckstellen auf der Haut des Patienten bilden. Beispielsweise kann der Quersteg 54 flächig oder netzartig ausgebildet sein. Zur Verbindung der verschiedenen Gurte und Stege miteinander sind diese jeweils an ihren Kontaktstellen vernäht und/oder verklebt oder mit sonstigen für Sicherheitsbandagen 100 bekannten Verbindungsmitteln miteinander verbunden.

Um eine Sicherheitsbandage 100 mit Schultergurten 24a, 24b nachzurüsten sind die Schultergurte 24a, 24b lösbar am Leibgurt 14 bzw. dem Haltegurt 10 befestigt, beispielsweise an weiteren Segelösen 17 des Leibgurts 14, und können auch als Nachrüstsatz für eine bereits vorhandene Sicherheitsbandage 100 angeboten werden. Die Querstege 54 können auch flächig ausgebildet sein bzw. netzförmig, damit nur ein geringfügiger Druck auf den Rücken des Patienten ausgeübt wird, um ihn nicht übermäßig zu beinträchtigen.

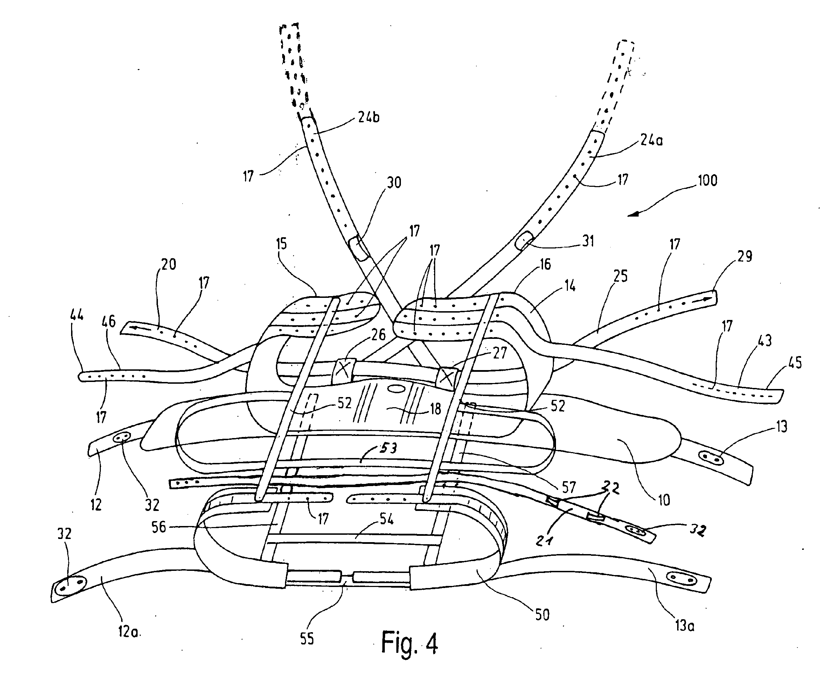

Aus Figur 4 geht eine weitere Ausführungsform der erfindungsgemäßen Sicherheitsbandage hervor. Bei dieser Ausführungsform sind die Schultergurte 24a, 24b verlängert. Zwischen Leibgurt 14 und Quersteg 55 ist eine Schultergurtfixierung 21 angeordnet, die um die Oberschenkel des Patienten führbar und verschließbar ist, insbesondere mit einem Gurtschloss, sowie Schlaufen 22 aufweist, durch die die Schultergurte 24a, 24b gesteckt werden können. Die Schlaufen 22 sind dabei als Taschen für Gurtschlösser derart ausgebildet, dass die Schultergurte 24a, 24b an diesen befestigbar sind.

### BEZUGSZEICHENLISTE:

- 100: Sicherheitsbandage
- 10: Haltegurt
- 12, 13: Ende
- 12a ,13a: Ende
- 14: Leibgurt
- 15, 16: Ende
- 17: Segelösen
- 18: Durchstecktasche
- 21: Schultergurtfixierung
- 22: Schlaufen
- 24a, 24b: Schultergurte
- 25: Gurt
- 26, 27: Schlaufe
- 28, 29: Ende
- 30: Schlaufe
- 31: Tasche
- 32: Öse
- 43, 44: Befestigungsgurte
- 45, 46: Ende
- 50: Oberschenkelmanschette
- 52: Längsgurt
- 53: zweiter Quersteg
- 54: erster Quersteg
- 55: weiterer Quersteg
- 56, 57: Beinhalterungsgurt
- 58: Taillengurt
- 59: Taillengurt

## Patentansprüche

1. Sicherheitsbandage (100) zur Begrenzung der Beweglichkeit eines auf einem Lager liegenden Patienten bzw. zur Positionierung eines Patienten auf einem Lager, mit einem Haltegurt (10), mit dem ein Leibgurt (14) beweglich verbunden ist, der geeignet ist, den Körper des Patienten im Bauchbereich zu umschtießen, , und mit zwei Oberschenkelmanschetten (50), die ausgebildet sind, um jeweils einen der Oberschenkel des Patienten zu umgreifen, wobei die Sicherheitsbandage (100) zwei quer zum Leibgurt angeordnete Beinhalterungsgurte (56, 57) aufweist, die den Leibgurt (14) und die Oberschenkelmanschetten (50) verbinden, **dadurch gekennzeichnet, dass** zwischen den Beinhalterungsgurten (56, 57) materiale Querstege (54, 55) vorgesehen sind.

2. Sicherheitsbandage nach Anspruch 1, **dadurch gekennzeichnet, dass** an einer Oberseite der Sicherheitsbandage (100) zwei Längsgurte (52) angeordnet sind, die jeweils mit einer Oberschenkelmanschette (50) verbunden sind und/oder für die Längsgurte (52) ein weiterer Quersteg (53) vorgesehen ist, der ebenfalls mit den Beinhalterungsgurten (56, 57) verbunden ist.

3. Sicherheitsbandage nach Anspruch 1 oder 2, **dadurch gekennzeichnet, dass** an der Sicherheitsbandage (100) Schultergurte (24a, 24b) vorgesehen sind, die am Leibgurt (14) fest oder lösbar angebracht sind und/oder der Leibgurt (14) mit einer Schulterhalterung fest oder lösbar verbunden ist und/oder die materialen Querstege (54, 55) aus Stoff oder Gewebe sind und/oder als Netz ausgebildet sind und/oder die Beinhalterungsgurte (56, 57) und die Längsgurte (52) lösbar am Leibgurt (14) befestigt sind.

4. Sicherheitsbandage nach Anspruch 3, **dadurch gekennzeichnet, dass** die Beinhalterungsgurte (56, 57) und die Schultergurte (24a, 24b) sowie die Längsgurte (52) einstückig ausgebildet sind, wobei die einstückig ausgebildeten Gurte (24a, 24b, 52, 56, 57) in eine Durchstecktasche (18), die sich an dem Leibgurt (14) befindet, durchsteckbar sind und/oder der einstückig ausgebildete Gurt (24a, 24b, 52, 56, 57) Segelösen und/oder Taschen und/oder Schlaufen aufweist und/oder die Sicherheitsbandage (100) einen Taillengurt (25) aufweist, der im Wesentlichen parallel zum Leibgurt (14) verläuft, wobei der Taillengurt (25) durch Schlaufen, die an den Schultergurten (24a, 24b) angeordnet sind, hindurch steckbar ist und/oder der einstückig ausgebildete Gurt (24a, 24b, 52, 56, 57) mit Schlössern am Leibgurt (14) sowie an den Oberschenkelmanschetten (50) befestigbar ist und/oder der einstückig ausgebildete Gurt (24a, 24b, 52, 221, 222) fest mit dem Leibgurt (14) vernäht ist.

5. Sicherheitsbandage nach einem der Ansprüche 2 bis 4, **dadurch gekennzeichnet, dass** die Enden (12a, 13a) an den Oberschenkelmanschetten (50), die als Betthalterung dienen, diagonal mit den Enden (12, 13) des Haltegurtes (10) verbindbar sind und/oder im oberen Oberschenkelbereich der zweite Quersteg (53) schließbar ist und die Oberschenkel des Patienten im oberen Bereich umgreift und/oder der Quersteg (53) über einen Verbindungsgurt schließbar ist, wobei an den offenen Enden Verschlussmittel angebracht sind, an denen der Verbindungsgurt lösbar befestigbar ist.

6. Sicherheitsbandage nach einem der Ansprüche 3 bis 5, **dadurch gekennzeichnet, dass** die Schulterhalterung mit den zwei Schultergurten (24a, 24b) versehen ist, die über die Schultern des Patienten verlaufen, sowie im mittleren Bereich eines Taillengurtes (25, 58, 59) mit dem Taillengurt (25, 58, 59) verbunden sind, wobei der Taillengurt (25, 58, 59) die Taille des Patienten umgreift und beide Enden des Taillengurtes (25, 58, 59) mit jeweils beiden Schultergurten (24a, 24b) - diese wiederum miteinander verbindend - lösbar miteinander verbunden sind, sowie Gurtschlösser, die mit einer mit geeigneten Verschlussmitteln zu öffnenden Verriegelungsposition versehen sind, die Enden des Taillengurtes (25, 58, 59) und die Schultergurte (24a, 24b) jeweils paarweise miteinander verbunden sind und/oder die Schultergurte (24a, 24b) an dem Taillengurt (25, 58, 59) über Schlaufen (26, 27) am Leibgurt (14) befestigt ist.

7. Sicherheitsbandage nach einem der Ansprüche 3 bis 6, **dadurch gekennzeichnet, dass** an den Schultergurten (24a, 24b) ein weiterer Brustgurt angeordnet ist und/oder die Schultergurte (24a, 24b) lösbar an der Sicherheitsbandage (100) befestigt sind und/oder der mindestens ein Quersteg (54) flächig ausgeführt ist und/oder die Beinhalterungsgurte (56, 57) Holmhaltegurte aufweisen, die die Oberschenkel des Patienten umgreifen und/oder die Holmhaltegurte Kanäle aufweisen, durch die Schultergurte einer Schulterhalterung durchsteckbar sind.

8. Sicherheitsbandage nach einem der vorhergehenden Ansprüche, **dadurch gekennzeichnet, dass** die Beinhalterungsgurte (56, 57) an einem Fußende des Bettrahmens lösbar befestigbar sind und/oder mindestens ein Verlängerungsgurt am Quersteg (53, 54, 55) und/oder an den Beinhalterungsgurten (56, 57) und/oder Haltgurt (10) und/oder Leibgurt (14) befestigt ist oder befestigbar ist und derart ausgestaltet ist, dass er an einem Fußende des Bettrahmens lösbar befestigbar ist.

9. Sicherheitsbandage nach einem der Ansprüche 3 bis 8, **dadurch gekennzeichnet, dass** an den/dem Schultergurten (24a, 24b) und/oder Haltegurt (10) und/oder Leibgurt (14) und/oder Querstegen (54) Verlängerungsgurte befestigt oder befestigbar sind, die am Kopfende des Bettrahmens lösbar befestigbar sind und/oder die Beinhalterungsgurte (56, 57) und die Schultergurte (24a, 24b) sowie die Längsgurte (52) einstückig ausgebildet sind, wobei die einstückig ausgebildeten Gurte (24 a, 24b, 52, 56, 57) in eine Durchstecklasche (18), die sich an dem Haltegurt (10) befindet, hindurch steckbar sind.

10. Sicherheitsbandage nach Anspruch 4, **dadurch gekennzeichnet, dass** der einstückig ausgebildete Gurt (24a, 24b, 56, 57) fest mit dem Haltegurt (10) vernäht ist.

11. Sicherheitsbandage nach einem der Ansprüche 1 bis 4, **dadurch gekennzeichnet, dass** die Enden (12a, 13a) an den Oberschenkelmanschetten (50), die als Betthalterung dienen, fest oder lösbar mit den Enden (12, 13) des Haltegurts (10) fest oder lösbar verbindbar sind.

12. Sicherheitsbandage nach einem der Ansprüche 3 bis 11, **dadurch gekennzeichnet, dass** zwischen Leibgurt (14) und Quersteg (55) eine Schultergurtfixierung (21) angeordnet ist, die um die Oberschenkel des Patienten führbar und umschließbar ist, insbesondere mit einem Gurtschloss, sowie Schlaufen (22) aufweist, durch die Schultergurte (24a, 24b) gesteckt werden können und/oder die Schlaufen als Taschen für Gurtschlösser ausgebildet sind, an denen die Schultergurte (24a, 24b) befestigbar sind.

13. Nachrüstsatz für eine Sicherheitsbandage (100) zur Begrenzung der Beweglichkeit eines auf einem Lager liegenden Patienten bzw. zur Positionierung eines Patienten auf einem lager, wobei die Sicherheitsbandage (100) einem fest auf das lager auflegbaren Haltegurt (10) aufweist, der mit einem Leibgurt (14) beweglich verbunden ist, der geeignet ist, den Körper des Patienten zu umschließen, wobei für den habtegurt (10) und für den Leibgurt (14) Verschlussmittel vorgesenhen sind, **dadurch gekennzeichnet, daß** der Nachrüstsatz zwei quer zum Leibgurt (14) anzuordnende Bein- haltungsgurte (56, 57) mit jeweils einer Oberschenkelmanschette (50) aufweist, wobei Beinhaltungsgurten (56, 57) materiale Querstege (54, 55) vorgesehen sind, und die Beinhaltungsgurte (56, 57) lösbar oder nicht lösbar an der Sicherheitsbandage (100) befestigbar sind.

## Claims

1. Safety bandage (100) for limiting the mobility of a patient lying on a bed and/or for the positioning of a patient on a bed, with a retaining belt (10) with which a body belt (14) which is suited for surrounding the body of the patient in the abdominal region is movably connected, and with two thigh cuffs (50) each of which is configured to surround one of the thighs of the patient, with the safety bandage (100) being provided with two leg-retaining belts (56, 57) arranged transversely to the body belt which connect the body belt (14) and the thigh cuffs (50), **characterised in that** material cross-links (54, 55) are provided between the leg-retaining belts (56, 57).

2. Safety bandage according to Claim 1 **characterised in that** at one upper side of the safety bandage (100) two longitudinal belts (52) are arranged with each of them being connected with a thigh cuff (50) and/or with another cross-link (53) which is also connected with the leg-retaining belts(56, 57) being provided for the longitudinal belts (52).

3. Safety bandage according to Claim 1 or 2 **characterised in that** shoulder straps (24a, 24b) are provided at the safety bandage (100) which are firmly or detachably fixed at the body belt (14) and/or that the body belt (14) is firmly or detachably connected with a shoulder holding device and/or the material cross-links (54, 55) are made of cloth or fabric and/or are formed as a web and/or the leg-retaining belts(56, 57) and the longitudinal belts (52) are detachably fixed at the body belt (14).

4. Safety bandage according to Claim 3 **characterised in that** the leg-retaining belts (56, 57) and the shoulder straps (24a, 24b) as well as the longitudinal belts (52) are integral where the belts that are integral (24a, 24b, 52, 56, 57) can be passed through into a pass-through pocket (18) which is located at the body belt (14) and/or the integral belt (24a, 24b, 52, 56, 57) is provided with eyelets and/or pockets and/or loops and/or the safety bandage (100) being provided with a waist belt (25) which runs essentially in parallel to the body belt (14) with the waist belt (25) being pushable through loops which are arranged at the shoulder straps (24a, 24b) and/or the integral belt (24a, 24b, 52, 56, 57) can be fixed with buckles at the body belt (14) as well as at the thigh cuffs (50) and/or the integral belt (24a, 24b, 52, 221, 222) is firmly sewn up with the body belt (14).

5. Safety bandage according to any of the Claims 2 to 4 **characterised in that** the ends (12a, 13a) at the thigh cuffs (50) which serve as a bed-holding device are diagonally connectable with the ends (12, 13) of the retaining belt (10) and/or in the upper thigh region the second cross-link (53) is lockable and surrounds the thighs of the patient in the upper region and/or the cross-link (53) is lockable via a connecting belt with locking means being provided at the open ends to which the connecting belt can be detachably fixed.

6. Safety bandage according to any of the Claims 3 to 5 **characterised in that** the shoulder-holding device is provided with the two shoulder straps (24a, 24b) which run across the shoulders of the patient and are connected in the central region of a waist belt (25, 58, 59) with the waist belt (25, 58, 59), with the waist belt (25, 58, 59) surrounding the waist of the patient and both ends of the waist belt (25, 58, 59) with both shoulder straps (24a, 24b) each - the same in turn interconnecting them - being detachably connected with each other, as well as belt buckles which are provided with a locking position that can be opened with suitable locking means, with the ends of the waist belt (25, 58, 59) and the shoulder straps (24a, 24b) being each interconnected in pairs and/or the shoulder straps (24a, 24b) at the waist belt (25, 58, 59) being fixed at the body belt (14) through loops (26, 27).

7. Safety bandage according to any of the Claims 3 to 6 **characterised in that** an additional breast belt is arranged at the shoulder straps (24a, 24b) and/or the shoulder straps (24a, 24b) are detachably fixed at the safety bandage (100) and/or the at least one cross-link (54) is arranged over a certain area and/or the leg-retaining belts (56, 57) are provided with bar-retaining belts which surround the thighs of the patient and/or the bar-retaining belts are provided with channels through which the shoulder straps of a shoulder-holding device may be pushed.

8. Safety bandage according to any of the preceding Claims **characterised in that** the leg-retaining belts (56, 57) are detachably fixable at a foot-end of the bed frame and/or at least one extension belt is fixed or fixable at the cross-link (53, 54, 55) and/or at the leg-retaining belts (56, 57) and/or the retaining belt (10) and/or body belt (14) and is configured such as to be detachably fixable at one foot-end of the bed frame.

9. Safety bandage according to any of the Claims 3 to 8 **characterised in that** at the shoulder straps (24a, 24b) and/or at the retaining belt (10) and or at the body belt (14) and/or at the cross-links (54) extension belts are fixed or fixable which are detachably fixable at the head-end of the bed frame and/or the leg-retaining belts (56, 57) and the shoulder straps (24a, 24b) as well as the longitudinal belts (52) are integral, where the integral belts (24a, 24b, 52, 56, 57) can be pushed through a push-through loop (18) which is provided at the retaining belt (10).

10. Safety bandage according to Claim 4 **characterised in that** the integral belt (24a, 24b, 56, 57) is firmly sewn up with the retaining belt (10).

11. Safety bandage according to any of the Claims 1 to 4 **characterised in that** the ends (12a, 13a) at the thigh cuffs (50) which serve as bed-holding device are firmly or detachably connectable with the ends (12, 13) of the retaining belt(10).

12. Safety bandage according to any of the Claims 3 to 11 **characterised in that** between the body belt (14) and the cross-link (55) a shoulder strap fixing device (21) is arranged which can be led around the patient's thighs and surround the same, in particular by means of a belt buckle and is provided with loops (22) through which the shoulder straps (24a, 24b) may be pushed and/or the loops are formed as pockets for belt buckles at which the shoulder straps (24a, 24b) can be fixed.

13. Retrofit kit for a safety bandage (100) for limiting the mobility of a patient lying on a bed and/or for the positioning of a patient on a bed, with the safety bandage (100) being provided with a retaining belt (10) which can be firmly placed onto the bed and is flexibly connected with a body belt (14) which is suited for surrounding the body of the patient, locking means being provided for the retaining belt (10) and for the body belt (14) **characterised in that** the retrofit kit is provided with two leg-retaining belts (56, 57) to be arranged transversely to the body belt (14) each with a thigh cuff (50) with material cross-links (54, 55) being provided between the leg-retaining belts (56, 57) and the leg-retaining belts (56, 57) are detachably or not detachably fixable at the safety bandage (100).

## Revendications

1. Bandage de sécurité (100) pour limiter la mobilité d'un patient couché sur un lit ou pour positionner un patient sur un lit, avec une sangle de retenue (10), à laquelle est reliée de manière mobile une sangle ventrale (14) qui est apte à entourer le corps du patient dans la région du ventre, et avec deux manchons pour cuisses (50) qui sont conçus pour s'appliquer chacun autour d'une des cuisses du patient, le bandage de sécurité (100) présentant deux sangles de retenue des jambes (56, 57) qui sont disposées transversalement à la sangle ventrale et qui relient la sangle ventrale (14) et les manchons pour cuisses (50), **caractérisé en ce que** des branches transversales de matériau (54, 55) sont prévues entre les sangles de retenue des jambes (56, 57).

2. Bandage de sécurité selon la revendication 1, **caractérisé en ce que** deux sangles longitudinales (52) sont disposées sur une face supérieure du bandage de sécurité (100), chacune des sangles longitudinales (52) étant reliée à un manchon pour cuisse (50), et/ou une branche transversale supplémentaire (53), également reliée aux sangles de retenue des jambes (56, 57), étant prévue pour les sangles longitudinales (52).

3. Bandage de sécurité selon la revendication 1 ou 2, **caractérisé en ce que** des sangles d'épaules (24a, 24b) fixées de manière fixe ou amovible à la sangle ventrale (14) sont prévues sur le bandage de sécurité (100), et/ou la sangle ventrale (14) est reliée de manière fixe ou amovible à un harnais d'épaules, et/ou les branches transversales de matériau (54, 55) sont en étoffe ou en tissu et/ou sont réalisées sous forme de maillage, et/ou les sangles de retenue des jambes (56, 57) et les sangles longitudinales (52) sont fixées de manière amovible à la sangle ventrale (14).

4. Bandage de sécurité selon la revendication 3, **caractérisé en ce que** les sangles de retenue des jambes (56, 57) et les sangles d'épaules (24a, 24b) ainsi que les sangles longitudinales (52) sont réalisées d'un seul tenant, les sangles réalisées d'un seul tenant (24a, 24b, 52, 56, 57) pouvant être passées à travers une poche de passage (18) qui se trouve sur la sangle ventrale (14), et/ou la sangle réalisée d'un seul tenant (24a, 24b, 52, 56, 57) présentant des oeillets et/ou des poches et/ou des boucles, et/ou le bandage de sécurité (100) présente une sangle de taille (25) qui s'étend sensiblement parallèlement à la sangle ventrale (14), la sangle de taille (25) pouvant être passée à travers des boucles qui sont disposées sur les sangles d'épaules (24a, 24b), et/ou la sangle réalisée d'un seul tenant (24a, 24b, 52, 56, 57) peut être fixée par des attaches sur la sangle ventrale (14) ainsi que sur les manchons pour cuisses (50), et/ou la sangle réalisée d'un seul tenant (24a, 24b, 52, 221, 222) est fixement reliée par couture à la sangle ventrale (14).

5. Bandage de sécurité selon l'une des revendications 2 à 4, **caractérisé en ce que** les extrémités (12a, 13a) des manchons pour cuisses (50), qui servent de fixation au lit, peuvent être reliées diagonalement aux extrémités (12, 13) de la sangle de retenue (10), et/ou la deuxième branche transversale (53) peut être fermée dans la région supérieure des cuisses et entoure les cuisses du patient dans la région supérieure, et/ou la branche transversale (53) peut être fermée au moyen d'une sangle de liaison, des moyens de fermeture étant fixés aux extrémités ouvertes, auxquels la sangle de liaison peut être fixée de manière amovible.

6. Bandage de sécurité selon l'une des revendications 3 à 5, **caractérisé en ce que** le harnais d'épaules est pourvu des deux sangles d'épaules (24a, 24b) qui passent sur les épaules du patient et qui sont reliées dans la région centrale d'une sangle de taille (25, 58, 59) à la sangle de taille (25, 58, 59), la sangle de taille (25, 58, 59) entourant la taille du patient et les deux extrémités de la sangle de taille (25, 58, 59) étant reliées de manière amovible entre elles respectivement par deux sangles d'épaules (24a, 24b) - ces dernières étant à leur tour reliées entre elles -, et d'attaches de sangle, qui sont dotées d'une position de verrouillage à ouvrir par des moyens de fermeture appropriés, les extrémités de la sangle de taille (25, 58, 59) et les sangles d'épaules (24a, 24b) étant respectivement reliées entre elles par paires, et/ou les sangles d'épaules (24a, 24b) étant fixées à la sangle de taille (25, 58, 59) au moyen de boucles (26, 27) sur la sangle ventrale (14).

7. Bandage de sécurité selon l'une des revendications 3 à 6, **caractérisé en ce qu'**une sangle de poitrine supplémentaire est disposée sur les sangles d'épaules (24a, 24b), et/ou les sangles d'épaules (24a, 24b) sont fixées de manière amovible au bandage de sécurité (100), et/ou la branche transversale au moins unique (54) est réalisée plane, et/ou les sangles de retenue des jambes (56, 57) présentent des sangles de retenue montantes qui entourent les cuisses du patient, et/ou les sangles de retenue montantes présentent des canaux à travers lesquels peuvent être passées des sangles d'épaules d'un harnais d'épaules.

8. Bandage de sécurité selon l'une des revendications précédentes, **caractérisé en ce que** les sangles de retenue des jambes (56, 57) peuvent être fixées de manière amovible à une extrémité de pied du cadre de lit, et/ou au moins une sangle de prolongement est fixée ou peut être fixée à la branche transversale (53, 54, 55) et/ou aux sangles de retenue des jambes (56, 57) et/ou à la sangle de retenue (10) et/ou à la sangle ventrale (14), et est configurée de telle sorte qu'elle peut être fixée de manière amovible à une extrémité de pied du cadre de lit.

9. Bandage de sécurité selon l'une des revendications 3 à 8, **caractérisé en ce que** des sangles de prolongement sont fixées ou peuvent être fixées aux sangles d'épaules (24a, 24b) et/ou à la sangle de retenue (10) et/ou à la sangle ventrale (14) et/ou aux branches transversales (54), sangles qui peuvent être fixées de manière amovible à une extrémité de pied du cadre de lit, et/ou les sangles de retenue des jambes (56, 57) et les sangles d'épaules (24a, 24b) ainsi que les sangles longitudinales (52) sont réalisées d'un seul tenant, les sangles réalisées d'un seul tenant (24a, 24b, 52, 56, 57) pouvant être passées à travers une poche de passage (18) qui se trouve sur la sangle de retenue (10).

10. Bandage de sécurité selon la revendication 4, **caractérisé en ce que** la sangle réalisée d'un seul tenant (24a, 24b, 52, 56, 57) est fixement reliée par couture à la sangle de retenue (10).

11. Bandage de sécurité selon l'une des revendications 1 à 4, **caractérisé en ce que** les extrémités (12a, 13a) des manchons pour cuisses (50), qui servent de fixation au lit, peuvent être reliées de manière fixe ou amovible aux extrémités (12, 13) de la sangle de retenue (10).

12. Bandage de sécurité selon l'une des revendications 3 à 11, **caractérisé en ce qu'**une fixation de sangle d'épaule (21) est disposée entre la sangle ventrale (14) et la branche transversale (55), fixation qui peut être dirigée autour des cuisses du patient et les entourer, en particulier avec une attache de sangle, et elle présente des boucles (22) à travers lesquelles peuvent être passées des sangles d'épaules (24a, 24b), et/ou les boucles sont réalisées sous la forme de poches pour des attaches de sangles auxquelles peuvent être fixées les sangles d'épaules (24a, 24b).

13. Kit de mise à niveau pour un bandage de sécurité (100) destiné à limiter la mobilité d'un patient couché sur un lit ou à positionner un patient sur un lit, le bandage de sécurité (100) présentant une sangle de retenue (10) pouvant être posée fixement sur le lit, qui est reliée de manière mobile à une sangle ventrale (14) apte à entourer le corps du patient, des moyens de fermeture étant prévus pour la sangle de retenue (10) et pour la sangle ventrale (14), **caractérisé en ce que** le kit de mise à niveau présente deux sangles de retenue des jambes (56, 57) à disposer transversalement à la sangle ventrale (14) et pourvues chacune d'un manchon pour cuisse (50), des branches transversales de matériau (54, 55) étant prévues entre les sangles de retenue des jambes (56, 57), et les sangles de retenue des jambes (56, 57) pouvant être fixées au bandage de sécurité (100) de manière amovible ou non amovible.
